# EUROPÄISCHE PATENTANMELDUNG

(11) **EP 2 347 752 A2**
(43) Veröffentlichungstag der Anmeldung: **27.07.2011**
(21) Anmeldenummer: 10160265.4
(22) Anmeldetag: 19.04.2010
(51) Int. Cl.: A61K 8/37, A61Q 5/04, A61Q 5/10

(54) **Haarfärbemittel und Haarwellmittel mit verbesserter Hautverträglichkeit**

(30) Priorität: 19.05.2009 DE 102009003226
(71) Anmelder: Henkel AG & Co. KGaA, 40589 Düsseldorf (DE)
(72) Erfinder: Kleen, Astrid, 20457, Hamburg (DE)

(57) **Zusammenfassung**

Gegenstand der Erfindung ist ein Mittel zur Farb- und/oder dauerhaften Formveränderung von keratinischen Fasern, dadurch gekennzeichnet, dass es in einem kosmetischen Träger mindestens eine farb- und/oder formverändernde Komponente und mindestens einen speziellen Fettsäureester enthält. Die erfindungsgemäßen Mittel eignen sich insbesondere zur Reduktion der Kopfhautirritation, die im Zusammenhang mit der oxidativen Haarbehandlung wie oxidative Haarfärbung, Haaraufhellung und Dauerwellbehandlung auftritt.

## Beschreibung

Gegenstand der Erfindung ist ein Mittel zur Farb- und/oder dauerhaften Formveränderung von keratinischen Fasern, welches in einem kosmetischen Träger mindestens eine farb- und/oder formverändernde Komponente sowie mindestens einen bestimmten Fettsäureester enthält. Die erfindungsgemäßen Mittel eignen sich insbesondere zur Reduktion der Haarschädigung und zur Verbesserung der Hautverträglichkeit. Weiterhin betrifft die Erfindung ein Verfahren zur Anwendung solcher Mittel auf keratinhaltigen Fasern. Darüber hinaus betrifft die Erfindung die Verwendung des Mittels zur Färbung und/oder dauerhaften Formveränderung mit verringertem Irritationspotential.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe und Formleistung möglichst minimale Schädigungen auf dem Haar hervorrufen und insbesondere eine gute Hautverträglichkeit aufweisen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich zwar durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese bilden dann im Rahmen oxidativer Prozesse im Haar naturanaloge Farbstoffe ausbilden.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Die dauerhafte Verformung von Keratinfasern wird üblicherweise so durchgeführt, dass man die Faser mechanisch verformt und die Verformung durch geeignete Hilfsmittel festlegt. Vor und/oder nach dieser Verformung behandelt man die Faser mit der wässrigen Zubereitung einer keratinreduzierenden Substanz, wobei diese einen Teil der Disulfid-Bindungen des Keratins zu ThiolGruppen spaltet, so dass es zu einer Lockerung der Peptidvernetzung und infolge der Spannung der Faser durch die mechanische Verformung zu einer Neuorientierung des Keratingefüges kommt, und spült nach einer Einwirkungszeit mit Wasser oder einer wässrigen Lösung. In einem zweiten Schritt behandelt man dann die Faser mit der wässrigen Zubereitung eines Oxidationsmittels, unter dessen Einfluss erneut Disulfid-Bindungen geknüpft werden, und so das Keratingefüge in der vorgegebenen Verformung neu fixiert wird. Nach einer Einwirkungszeit wird auch dieses ausgespült und die Faser von den mechanischen Verformungshilfsmitteln (Wickler, Papilloten) befreit. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Färbemittel und Dauerwellmittel enthalten daher in der Regel Wasserstoffperoxid als Oxidationsmittel oder Fixiermittel. Dies führt neben dem gewünschten kosmetischen Effekt zu oxidativen Schädigungen an der Haarstruktur und zu einem erhöhten Irritationspotential der Kopfhaut. Auch der üblicherweise in solchen Mitteln basisch eingestellte pH-Wert führt zur Erhöhung des Irritationspotentials. Besonders Anwender mit empfindlicher Haut können auf solche Mittel mit der Ausbildung von Entzündungen und mit Schmerzgefühl reagieren. Daher sind solche Produkte insbesondere bei Personen mit Neigung zur Ausbildung von Allergien nur bedingt einsetzbar. Erschwerend kommt hinzu, dass einige der häufig eingesetzten kosmetischen Inhaltsstoffe ein nicht unerhebliches Allergiepotential tragen. Dies gilt sowohl für Oxidationsfarbstoffvorprodukte, wie auch für bestimmte Parfümstoffe, bestimmte UV-Filtersubstanzen oder bestimmte Konservierungsmittel.

Aufgabe der vorliegenden Erfindung ist daher die Bereitstellung kosmetischer Mittel zur Farb- und/oder Formveränderung keratinischer Fasern, die eine verbesserte Hautverträglichkeit aufweisen. Es ist insbesondere Aufgabe der vorliegenden Anmeldung, das Irritationspotential für die Haut zu senken und so eine Minimierung des Allergiepotentials zu erzielen. Weiterhin ist es Aufgabe der vorliegenden Erfindung, ein Mittel bereitzustellen, welches den Einsatz von erhöhten Mengen an Oxidationsfarbstoffvorprodukten und/oder Oxidationsmittel erlauben, so dass die Einwirkzeit dieser Mittel verkürzt werden kann, ohne dass die erhöhte Konzentration dieser Reizstoffe zu Irritationen und Reizungen der Kopfhaut führt.

In nicht vorhersehbarer Weise konnte nun gefunden werden, dass farb- und/oder formverändernde Mittel, die neben einer farb- und/oder formverändernden Komponente mindestens spezielle Fettsäureester enthalten, die oben genannten Nachteile vermeiden. Bedingt durch die Schutzwirkung bei Anwendung des erfindungsgemäßen Mittels kann die Hautverträglichkeit hierdurch deutlich verbessert werden.

Ein erster Gegenstand der Erfindung ist daher ein Mittel zur Farb- und/oder Formveränderung von keratinischen Fasern, insbesondere menschlichen Haaren, welches in einem kosmetischen Träger mindestens eine farb- und/oder formverändernde Komponente enthält, und welches dadurch gekennzeichnet ist, dass das Mittel zusätzlich mindestens einen Ester (a) aus einer C₁₀-C₂₀-Fettsäure und einem C₆-C₂₂-Fettalkohol und mindestens einen Ester (b) aus einer C₁₀-C₂₀-Fettsäure und einem C₆-C₁₂-Alkylpolyol enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als wesentlichen Inhaltsstoff enthält das erfindungsgemäße Mittel mindestens einen Ester (a) aus einer C₁₀-C₂₀-Fettsäure und einem C₆-C₂₂-Fettalkohol und mindestens einen Ester (b) aus einer C₁₀-C₂₀-Fettsäure und einem C₆-C₁₂-Alkylpolyol.

Unter einer C₁₀-C₂₀-Fettsäure sind gesättigte und ungesättigte, gegebenenfalls verzweigte und/oder substituierte Fettsäuren zu verstehen. Erfindungsgemäß geeignete Fettsäuren sind Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure oder Gadoleinsäure sowie deren technischer Mischungen wie beispielsweise Kokos-, Palm-, Palmkern- oder Talgfettsäure.

In einer bevorzugten Ausführungsform der vorliegenden Erfindung handelt bei den Fetttsäureestern (a) und (b) jeweils um Ester von mindestens einer ungesättigten C₁₀-C₂₀-Fettsäure sowie von mindestens einer gesättigten C₁₀-C₂₀-Fettsäure.

Dabei sind als Fettsäurekomponente der erfindungsgemäßen Ester solche Gemische von Fettsäuren besonders geeignet, die pflanzlichen Ursprungs und einen Gehalt an ungesättigten Fettsäuren von mindestens 50 Gew.-%, bezogen auf das Gesamtgewicht des natürlichen Fettsäuregemischs, bevorzugt von mindestens 60 Gew.-% und insbesondere bevorzugt von mindestens 70 Gew.-% aufweisen. Ein erfindungsgemäß bevorzugtes, pflanzliches Fettsäuregemisch ist das Fettsäuregemisch, welches aus Olivenöl gewonnen wird. Das Fettsäuregemisch aus Olivenöl enthält, je nach Ursprung und Behandlung, ca. 72 bis 80 Gew.-% Ölsäure, ca. 3 bis 11 Gew.-% Palmitinsäure, ca. 7 bis 8 Gew.-% Linolsäure, ca. 0 bis 14 Gew.-% Myristinsäure, ca. 0 bis 3 Gew.-% Stearinsäure sowie ca.0 bis 5 Gew.-% Palmitoleinsäure.

Die Ester des Fettsäuregemisches aus Olivenöl werden auch als Olivate bezeichnet. Eine bevorzugte Ausführungsform dieses Erfindungsgegenstands ist **dadurch gekennzeichnet, dass** das Mittel als Ester (a) und (b) jeweils ein Gemisch aus Estern von C₁₀-C₂₀-Fettsäuren, welches aus Olivenöl gewonnen wird, enthält.

Die Alkoholkomponente der Fettsäureester (a) und (b) ist einerseits ein C₆-C₂₂-Fettalkohol sowie andererseits ein C₆-C₁₂-Alkylpolyol.

Bei einem C₆-C₂₂-Fettalkohol im Sinne der Erfindung handelt es sich um einen Monohydroxyalkohol, welcher einen gesättigten oder ungesättigten Kohlenstoffrest mit einem Grundkörper aus 6 bis 22 Kohlenstoffatomen aufweist, und welcher gegebenenfalls verzweigt sein kann. Bevorzugt wird der Fettalkohol ausgewählt aus der Gruppe, die gebildet wird aus Capronalkohol, Caprylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Elaeostearylalkohol, Arachylalkohol, Gadoleylalkohol, Behenylalkohol, Erucylalkohol und Brassidylalkohol. Weiterhin kann als Fettalkohol ein Gemisch von Fettalkoholen unterschiedlicher Kohlenstoffkette eingesetzt werden, insbesondere wenn es sich um Fettalkohole natürlichen Ursprungs handelt. Besonders bevorzugt wird als Fettalkohol ein Fettalkoholgemisch, ausgewählt aus Talgfettalkohol, Cocosfettalkohol, Palmfettalkohol und Cetearylalkohol, eingesetzt.

Eine weitere Ausführungsform des ersten Erfindungsgegenstands ist daher dadurch gekennzeichnet, dass das Mittel als Ester (a) mindestens einen Ester einer C₁₀-C₂₀-Fettsäure mit einem C₆-C₂₂-Fettalkohol enthält, worin der besagte Fettalkohol ausgewählt ist aus der Gruppe, die gebildet wird aus Capronalkohol, Caprylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Elaeostearylalkohol, Arachylalkohol, Gadoleylalkohol, Behenylalkohol, Erucylalkohol und Brassidylalkohol sowie den Fettalkoholgemischen Talgfettalkohol, Cocosfettalkohol, Palmfettalkohol und Cetearylalkohol.

Besonders vorteilhaft wird erfindungsgemäß als Ester (a) ein Gemisch aus Estern von C₁₀-C₂₀-Fettsäuren, welches aus Olivenöl gewonnen wird, mit Cetearylalkohol eingesetzt (Cetearylolivat; INCI-Bezeichnung: Cetearyl Olivate).

Bei einem C₆-C₁₂-Alkylpolyol im Sinne der Erfindung handelt es sich um einen Polyhydroxyalkohol, welcher in seiner Struktur einen Grundkörper aus 6 bis 12 Kohlenstoffatomen sowie zwei oder mehr Hydroxygruppen trägt. Bevorzugt handelt es sich dabei um einen Polyol mit 6 Kohlenstoffatomen, insbesondere um einen C₆-Zuckeralkohol, bevorzugt abgeleitet von einer Hexose (Hexitol), sowie um Derivate auf Basis eines C₆-Zuckeralkohols. Besonders geeignete Hexitole sind erfindungsgemäß Galactitol, Mannitol und Sorbitol (auch Sorbit, Glucit oder Glucitol), besonders bevorzugt jeweils in der natürlichen D-Konfiguration. Weiterhin besonders vorteilhaft als Polyol-Komponente ist Sorbitan einzusetzen, welches sich nach Verlust eines Wassermoleküls und Ringschluss von Sorbitol ableitet. Eine weitere Ausführungsform der vorliegenden Erfindung ist daher **dadurch gekennzeichnet, dass** das Mittel als Ester (b) mindestens einen Ester einer C₁₀-C₂₀-Fettsäure mit einem C₆-C₁₂-Alkylpolyol enthält, worin der besagte Polyol ausgewählt ist aus der Gruppe, die gebildet wird aus den Hexitolen Galactitol, Mannitol und Sorbitol sowie aus Sorbitan.

Besonders vorteilhaft wird erfindungsgemäß als Ester (b) ein Gemisch aus Estern von C₁₀-C₂₀-Fettsäuren, welches aus Olivenöl gewonnen wird, mit Sorbitan eingesetzt (Sorbitanolivat; INCI-Bezeichnung: Sorbitan Olivate).

Ganz besonders vorteilhaft ist die Kombination der Fettsäureester Cetearylolivat und Sorbitanolivat. Erfindungsgemäß besonders bevorzugte Mittel sind daher **dadurch gekennzeichnet, dass** das Mittel als Ester ein Gemisch aus mindestens Cetearylolivat und mindestens Sorbitanolivat enthält.

Ein Handelsprodukt, welches die erfindungsgemäße Kombination aus Cetearylolivat und Sorbitanolivat (INCI-Bezeichnungen: Cetearyl Olivate and Sorbitan Olivate) umfasst, wird unter dem Handelsnamen Olivem 1000 Crystal Skin von der Firma B&TCompany vertrieben.

Eine Ausführungsform der vorliegenden Erfindung ist **dadurch gekennzeichnet, dass** das Mittel das Gemisch der Ester aus C₁₀-C₂₀-Fettsäure(n) und C₆-C₂₂-Fettalkohol(en) sowie C₆-C₁₂-Alkylpolyol(en) in einer Gesamtmenge von 0,0001 bis 5 Gew.-%, insbesondere von 0,001 bis 3,0 Gew.-%, jeweils bezogen auf das Gesamtgewicht des anwendungsbereiten Mittels, enthält.

Es hat sich gezeigt, dass das erfindungsgemäße Gemisch aus mindestens einen Ester einer C₁₀-C₂₀-Fettsäure mit einem C₆-C₂₂-Fettalkohol und mindestens einen Ester einer C₁₀-C₂₀-Fettsäure mit einem C₆-C₁₂-Alkylpolyol sich hervorragend dazu geeignet ist, die Schutzschicht in der Haut wiederherzustellen und somit sowohl Wasserverlust und Austrocknung vorzubeugen wie auch das Eindringen von Schadstoffen in die Haut zu verhindern. Im Besonderen lässt sich mit dieser Kombination die Integrität der Haut als Schutzschicht gegen Allergene erhalten und verbessern. Dabei wird die trans-epidermale Penetration von Allergenen in den stratum corneum verhindert, wodurch Reizungen und Entzündungsreaktionen in der Haut reduziert werden. Darüber hinaus ist diese Kombination geeignet, die zelluläre Produktion von Interleukin-1 alpha (IL-1α) als eines der für Entzündungen hauptverantwortlichen Cytokine zu inhibieren.

Dadurch wird insgesamt die Toleranzschwelle der Haut, auf irritierende Substanzen mit Reizung und Entzündungen zu reagieren, herabgesenkt. Somit werden sowohl Entzündungspotential wie auch Schmerzempfindung der Haut, insbesondere der Kopfhaut, reduziert. Außerdem kann sich angegriffene, empfindliche Haut beruhigen und so potentiell allergienauslösende Substanzen besser tolerieren. Insgesamt führt die Verwendung der erfindungsgemäßen Mittel zur Farb- und/oder Formveränderung von menschlichen Haaren zu einer Verbesserung der Hautverträglichkeit und zu einer Verringerung an Irritationen auf der Kopfhaut.

Die erfindungsgemäßen Mittel werden besonders vorteilhaft zur Farbveränderung keratinischer Fasern eingesetzt. Erfindungsgemäß bevorzugte Mittel sind daher **dadurch gekennzeichnet, dass** das Mittel als farbverändernde Komponente mindestens ein Oxidationsfarbstoffvorprodukt und/oder mindestens einen direktziehenden Farbstoff und/oder mindestens eine Vorstufe naturanaloger Farbstoffe und/oder mindestens Oxofarbstoffvorprodukte und/oder mindestens ein Aufhellmittel enthält.

In einer ersten Ausführungsform werden die farbverändernden Komponenten ausgewählt aus Oxidationsfarbstoffvorprodukten.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Erfindungsgemäße Entwicklerkomponenten werden ausgewählt aus p-Phenylendiamin, zweikernigen Entwicklerkomponenten, p-Aminophenol, o-Aminophenol, heterocyclischen Entwicklerkomponenten und/oder den Derivaten vorstehender Substanzklassen. Die Entwicklerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]-tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder einem der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-[(2-hydroxyethyl)aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol oder einem der physiologisch verträglichen Salze dieser Verbindungen.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze.

Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]-ethanol, 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylaminobenzol, 3-Ethylamino-4-methylphenol, 2,4-Dichlor-3-aminophenol und den physiologisch verträglichen Salzen der vorstehend genannten Verbindungen.

Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen der genannten Verbindungen.

Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen der genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Um das Irritationspotential der erfindungsgemäßen Mittel weiter zu senken, ist es erfindungsgemäß bevorzugt, die Oxidationsfarbstoffvorprodukte aus Farbstoffvorprodukten mit überwiegend geringem Irritationspotential auszuwählen. Insbesondere bevorzugt werden die Oxidationsfarbstoffvorprodukte daher ausgewählt aus der Gruppe, die gebildet wird aus 1,4-Diamino-2-(2-hydroxyethyl)benzol 1,5-Dihydroxynaphthalin, 2,7-Dihydroxynaphthalin, 4-Methylaminophenol, 2,4-Diaminophenoxyethanol Hydrochlorid, 3-Amino-2,4-dichlorphenol, 2-Methylresorcin, 2,4,5,6-Tetraaminopyrimidin, 1,3-Bis-(2,4-diaminophenoxy)propan, 3-Amino-2-chlor-6-methylphenol, 5-Amino-4-chlor-2-methylphenol Hydrochlorid, 2-Amino-3-hydroxypyridin, 2,6-Bis-(2-hydroxyethylamino)-1-methylbenzol Hydrochlorid, Bis-(2-hydroxy-5-aminophenyl)methan Hydrochlorid, N-(4-Amino-3-methylphenyl)-N-[3-(1 H-imidazol-1-yl)propyl]amin.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Zuge der Untersuchungen zu dieser Erfindung hat sich herausgestellt, dass durch die Verwendung von mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol in oxidativen Färbemitteln eine erhöhte Menge an Oxidationsfarbstoffvorprodukten einsetzbar ist, ohne dass Irritationen an der Kopfhaut für den Anwender auftreten. Durch den Einsatz höherer Konzentrationen an Farbstoffvorprodukten lässt sich jedoch die Einwirkzeit für den Anwender signifikant verkürzen. Dies hat neben einer Verbesserung des Anwendungskomforts zur Folge, dass weitere zeitabhängige Nachteile der oxidativen Färbung, wie zum Beispiel Schädigungen der Haarstruktur durch Oxidationsmittel oder Alkalisierungsmittel, reduziert werden.

Eine weitere Ausführungsform der vorliegenden Erfindung ist daher **dadurch gekennzeichnet, dass** das Mittel als farbverändernde Komponente mindestens ein Oxidationsfarbstoffvorprodukt enthält, wobei der Gewichtsanteil aller Oxidationsfarbstoffvorprodukte zwischen 2,0 und 25,0 Gew.-%, bevorzugt zwischen 3,0 und 20,0 Gew.-% und insbesondere zwischen 4,0 und 15,0 Gew.-%, jeweils bezogen auf das Gesamtgewicht des anwendungsbereiten Mittels, liegt.

Weiterhin können die erfindungsgemäßen Mittel als farbverändernde Komponente mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Um das Irritationspotential der erfindungsgemäßen Mittel weiter zu senken, ist es erfindungsgemäß bevorzugt, die direktziehenden Farbstoffe aus Farbstoffen mit überwiegend geringem Irritationspotential auszuwählen. Insbesondere bevorzugt werden direktziehenden Farbstoffe daher ausgewählt aus der Gruppe, die gebildet wird aus Basic Brown 17, Basic Brown 16, Basic Violet 2, Basic Yellow 57, Basic Yellow 87, Basic Red 76, Basic Blue 99, Acid Orange 7, Acid Yellow 1, Acid Yellow 3, Acid Yellow 23, Acid Black 1, Acid Red 33, Acid Red 52, Acid Blue 9, HC Yellow 2, HC Yellow 13, HC Orange 1, HC Red 3, HC Red 7, HC Red 10, HC Red 11, HC Blue 2, HC Blue 12, Disperse Violet 1, N,N'-Bis-(2-hydroxyethyl)-2-nitro-1,4-diaminobenzol, N-(2-Hydroxyethyl)-2-nitro-4-methylanilin, 4-(3-Hydroxypropyl)amino-3-nitrophenol, 4-Amino-2'-carboxyl-2-nitro-diphenylamin. In einer weiteren Ausführungsform der vorliegenden Erfindung wird die farbverändernde Komponente aus Farbstoffvorstufen naturanaloger Farbstoffe ausgewählt.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure sowie insbesondere 5,6-Dihydroxyindol.

Eine weitere Möglichkeit zur Farbgebung bietet die Verwendung von Färbemitteln, welche sogenannte Oxofarbstoffvorprodukte enthalten. Als erfindungsgemäße farbverändernde Komponente können daher in einer weiteren Ausführungsform auch Oxofarbstoffvorprodukte eingesetzt werden. Oxofarbstoffvorprodukte werden bevorzugt als Kombination aus mindestens einer Verbindung (Oxo1), die mindestens eine reaktive Carbonylgruppe enthält, mit mindestens einer Verbindung (Oxo2), ausgewählt aus C,H-aciden Verbindungen und/oder aus Verbindungen mit primärer oder sekundärer Aminogruppe oder Hydroxygruppe, eingesetzt.

Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in dem nichtoxidativen Prozess der sogenannten Oxofärbung die eigentlichen Farbstoffe aus. Die resultierenden Färbungen besitzen teilweise Farbechtheiten auf der keratinhaltigen Faser, die mit denen der Oxidationsfärbung vergleichbar sind.

Es ist jedoch erfindungsgemäß bevorzugt, wenn (Oxo2) nur unter C,H-aciden Verbindungen ausgewählt werden. Die voranstehend genannten Verbindungen (Oxo1) sowie (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

Es ist nicht erforderlich, dass Oxidationsfarbstoffvorprodukte, direktziehende Farbstoffe, Oxofarbstoffvorprodukte oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

In einer weiteren Ausführungsform der vorliegenden Erfindung wird die farbverändernde Komponente aus Aufhellmitteln ausgewählt. Solche Aufhellmittel sind üblicherweise chemische Oxidationsmittel, mit deren Hilfe natürliche und künstliche Farbstoffe im Haar zerstört und das Haar damit gebleicht wird. Als Aufhellmittel kommen daher Persulfate, Peroxomonosulfate, Chlorite, Hypochlorite und insbesondere Wasserstoffperoxid oder dessen Anlagerungsprodukte an Harnstoff, Melamin sowie Natriumborat in Frage.

Der Aufhelleffekt kann auch neben einer Färbung gewünscht sein. Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Es ist daher bevorzugt, wenn das Mittel zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die Anlagerungsprodukte an Harnstoff, Melamin, Polyvinylpyrrolidinon sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen **dadurch gekennzeichnet, dass** die fließfähige Oxidationsmittelzubereitung, bezogen auf ihr Gewicht, 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Soll helles oder aufgehelltes Haar bei der Behandlung eine Auffrischung des Aufhelleffekts erfahren, so kann es vorteilhaft sein, Wasserstoffperoxid und /oder eines seiner festen Anlagerungsprodukte in ein Shampoo oder ein ähnliches Behandlungsmittel einzuarbeiten. Damit wird jedoch in der Regel nur eine geringe Aufhellung erzielt. Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und anorganischen Persulfaten eingesetzt, woraus eine Steigerung des Aufhellvermögens der Mittel resultiert. Bevorzugte Persulfatsalz sind Ammoniumperoxodisulfat, Kaliumperoxodisulfat und Natriumperoxodisulfat.

Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei die Kombination aus mindestens einem Ester (a) einer C₁₀-C₂₀-Fettsäure mit einem C₆-C₂₂-Fettalkohol und mindestens einem Ester (b) einer C₁₀-C₂₀-Fettsäure mit einem C₆-C₁₂-Alkylpolyol in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten die Kombination der besagten Ester.

Wird eine starke Aufhellung gewünscht, so ist es erfindungsgemäß bevorzugt, wenn zusätzlich eine Blondierzubereitung, enthaltend mindestens ein anorganisches, der Oxidationsmittelzubereitung vor Vermischung mit der erfindungsgemäßen Färbezubereitung beigemischt wird.

Im Rahmen einer bevorzugten Ausführungsform handelt es sich bei den erfindungsgemäßen Mittel um dauerhaft formverändernde Mittel. Diese Mittel bestehen üblicherweise aus zwei beziehungsweise drei Zubereitungen, die nacheinander auf die Fasern aufgebracht werden. Im Weiteren werden folgende Bezeichnungen verwendet:
- "Wellmittel" für die wässrige Zubereitung der keratinreduzierenden Substanz,
- "Zwischenspülung" für die erste Spülung und
- "Fixiermittel" für die wässrige Zubereitung des Oxidationsmittels.

Obwohl die erfindungsgemäße Kombination der Fettsäureester (a) und (b) prinzipiell in jeder der genannten Zubereitungen eingesetzt werden kann, hat es sich als erfindungsgemäß besonders bevorzugt erwiesen, wenn sie im Wellmittel enthalten ist.

Die erfindungsgemäßen Wellmittel enthalten zwingend mindestens ein keratinreduzierende Substanz als formverändernde Komponente, vorzugsweise Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet als formverändernde Komponenten sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammoniumcarbonate und -hydrogencarbonate oder organische Amine wie Monoethanolamin.

Weiterhin können die erfindungsgemäßen Welllotionen wellkraftverstärkende Komponenten enthalten, bevorzugt ausgewählt aus heterocyclischen Verbindungen wie Imidazol, Pyrrolidin, Piperidin, Dioxolan, Dioxan, Morpholin, Piperazin sowie Derivate dieser Verbindungen, insbesondere 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin, Biotin, Hydantoin und Benzimidazol, wobei Imidazol besonders bevorzugt ist; Aminosäuren wie insbesondere Arginin, Citrullin, Histidin, Ornithin, Lysin, Oligopeptiden aus durchschnittlich 2-3 Aminosäuren, die einen hohen Anteil (> 50 %, insbesondere > 70 %) an den genannten Aminosäuren haben, sowie deren Salze, bevorzugt Arginin sowie dessen Salze und Arginin-reiche Oligopeptide; Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol, wobei 1,3-Diole, insbesondere 2-Ethyl-1,3-hexandiol und 1,3-Butandiol besonders bevorzugt sind. Die wellkraftverstärkenden Verbindungen können in den erfindungsgemäßen Welllotionen in Mengen von 0,5 bis 5 Gew.-%, bezogen auf die gesamte Welllotion, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Zwingender Bestandteil der erfindungsgemäßen Fixiermittel sind Oxidationsmittel, z. B. Natriumbromat, Kaliumbromat, Wasserstoffperoxid, und die zur Stabilisierung wässriger Wasserstoffperoxidzubereitungen üblichen Stabilisatoren. Erfindungsgemäß einsetzbare wässriger H₂O₂-Zubereitungen enthalten etwa 0,5 bis 15 Gew.-%, gebrauchsfertig in der Regel etwa 0,5 bis 3 Gew.-%, Wasserstoffperoxid. Der pH-Wert solcher wässriger H₂O₂-Zubereitungen liegt bevorzugt bei 2 bis 6, insbesondere 2 bis 4 und wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere Wasserstoffperoxid, enthalten.

Die erfindungsgemäßen Wellmittel beziehungsweise Fixiermittel sind üblicherweise einphasig formuliert, eingeschlossen von diesem Begriff sind dabei Systeme, die eine kontinuierliche Phase aufweisen, wie beispielsweise reine Öl-in-Wasser- oder Wasser-in-Öl-Emulsionen. Es hat sich gezeigt, das erfindungsgemäß auch Zwei- und Mehrphasensysteme bevorzugt sind. Dies sind Systeme, bei denen mindestens zwei separate, kontinuierliche Phasen vorliegen.

Weiterhin hat es sich als vorteilhaft erweisen, wenn die Oxidationsmittelzubereitungen aus Oxidationsfärbemitteln, Aufhellmitteln und/oder Fixiermitteln von formverändernden Mitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta-(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Unabhängig davon, ob es sich bei dem erfindungsgemäßen Mittel um ein Farbveränderungsmittel oder ein Formveränderungsmittel handelt, ist es erfindungsgemäß bevorzugt, dass das Mittel zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die festen Anlagerungsprodukte an Harnstoff, Melamin, Polyvinylpyrrolidinon sowie Natriumborat in Frage.

Hinsichtlich der Mengen an Wasserstoffperoxid im anwendungsbereiten Mittel wird auf die voranstehenden, je nach Anwendungsgebiet bevorzugten Mengen verwiesen.

Die erfindungsgemäßen Mittel enthalten weitere Hilfs- und Zusatzstoffe. Es hat sich weiterhin herausgestellt, dass sich die Hautirritation durch Haarfärbe- und/oder Haarwellmittel durch den Zusatz bestimmter Pflegestoffe zusätzlich unerwartet deutlich reduzieren lässt.

Als erfindungsgemäß besonders vorteilhaften zusätzlichen Pflegestoff hat sich dabei der Zellschutzstoff Ectoin ((*4S*)-1,4,5,6-Tetrahydro-2-methyl-pyrimidine-4-carboxylic acid; INCI-Bezeichnung: Ectoin) erwiesen. Eine weiterhin bevorzugte Ausführungsform der vorliegenden Erfindung ist daher dadurch gegeben, dass das Mittel zusätzlich Ectoin enthält. Bevorzugt enthält das erfindungsgemäße Mittel Ectoin zu 0,0001 bis 3,0 Gew.-%, bevorzugt zu 0,001 bis 1,0 Gew.-%, jeweils bezogen auf das Gesamtgewicht des anwendungsbereiten Mittels.

In einer bevorzugten Ausführungsform der Einfindung enthalten die Haarbehandlungsmittel, bezogen auf ihr Gewicht, neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% D-Panthenol.

In einer weiteren bevorzugten Ausführungsform der Einfindung enthalten die Haarbehandlungsmittel, bezogen auf ihr Gewicht, neben der erfindungsgemäßen Wirkstoffkombination 0,01 bis 2 Gew.-%, bevorzugt 0,05 bis 1,5 Gew.-% und insbesondere 0,1 bis 1 Gew.-% an Glycin, Serin und/oder Arginin.

Erfindungsgemäß ganz besonders bevorzugt aufgrund ihrer haarkräftigenden Wirkung sowie ihrer unterstützenden Wirkung in Bezug auf die Haarfülle sind die Extrakte aus Grünem Tee, Kamille, Mandel und Moringa. Insbesondere bevorzugt ist erfindungsgemäß ein Moringa-Extrakt, beispielsweise ein unter dem Handelsnamen Puricare^{®} LS 9658 oder Moringa Oleifera^{®} vertriebenes Produkt. In einer weiteren bevorzugten Ausführungsform der Einfindung enthalten die Haarreinigungs- und/oder -konditioniermittel, bezogen auf ihr Gewicht, neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% Moringa-Extrakt.

Diese Pflegestoffe können einzeln, bevorzugt jedoch in Kombinationen untereinander im erfindungsgemäßen Mittel eingesetzt werden. Eine weitere Ausführungsform der vorliegenden Erfindung ist daher **dadurch gekennzeichnet, dass** das Mittel als zusätzlichen Haarpflegestoff mindestens einen Pflegestoff, ausgewählt aus der Gruppe, gebildet aus D-Panthenol, Glycin, Serin, Arginin, Moringa Oleifera und/oder Ectoin, enthält.

Vorzugsweise stellen die anwendungsbereiten Mittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und weiteren nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe.
Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Der Zusatz im erfindungsgemäßen Mittel bewirkt eine hervorragende Avivage, insbesondere eine verbesserte Nasskämmbarkeit.

Als besonders vorteilhaft haben sich außerdem oberflächenaktive Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30, sowie Alkylpolyglykoside entsprechend der allgemeinen Formel RO-(Z)ₓ, wobei R für Alkyl, Z für Zucker sowie x für die Anzahl der Zuckereinheiten steht. Erfindungsgemäß bevorzugte Mittel enthalten als nichtionisches Tensid das Anlagerungsprodukt von CocosFettalkohol an (Poly-)glucose, welches unter der INCI-Bezeichnung Coco-Glucoside bekannt ist. Die anionischen, zwitterionischen, amphoteren und weiteren nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdickungsmittel um ein anionisches, synthetisches Polymer. Bevorzugte anionische Polymere sind Acrylsäure- und/oder Methacrylsäure-Polymere oder - Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogene Monomereinheiten, bevorzugt Acrylamid, Methacrylamid, Acrylsäure-C₁-C₄-Alkylester und Methacrylsäure-C₁-C₄-Alkylester, eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der Erfindung erwiesen. Insbesondere Tone, vorzugsweise Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Es hat sich zur weiteren Verringerung des Irritationspotentials der erfindungsgemäßen Mittel als vorteilhaft herausgestellt, wenn die Mittel frei sind von organischen UV-Lichtschutzfiltersubstanzen. Unter UV-Lichtschutzfiltern sind organische Substanzen zu verstehen, die in der Lage sind, ultraviolette Strahlen zu absorbieren und die aufgenommene Energie in Form längerwelliger Strahlung, z. B. Wärme wieder abzugeben. Bevorzugt enthält das erfindungsgemäße Mittel keine UV-Filtersubstanzen aus den folgenden Substanzklassen: 3-Benzylidencampher und dessen Derivate, z. B. 3-(4-Methylbenzyliden)campher; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene); Ester der Salicylsäure, vorzugsweise Salicylsäure-2-ethylhexylester, Salicylsäure-4-isopropylben-zylester, Salicylsäurehomomenthylester; Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyltriazon; Propan-1,3-dione, wie z.B. 1-(4-*tert*-Butylphenyl)-3-(4'-methoxyphenyl)propan-1,3-dion oder 1-Phenyl-3-(4'-isopropylphenyl)-propan-1,3-dion; 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze. In einer weiteren Ausführungsform der vorliegenden Erfindung sind die erfindungsgemäßen Mittel frei von UV-Lichtschutzfiltern, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenzimidazol-5-sulfonsäuren, Benzophenonsulfonsäuren und Benzoylmethanderivaten. Frei von UV-Lichtschutzfiltern im Sinne der vorliegenden Erfindung bedeutet, dass die anwendungsbereiten Mittel weniger als 0,05 Gew.-%, bevorzugt weniger als 0,005 Gew.-% und ganz besonders bevorzugt weniger als 0,0001 Gew.-% der UV-Lichtschutzfilter, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenzimidazol-5-sulfonsäuren, Benzophenonsulfonsäuren und Benzoylmethanderivaten, enthalten.

Weiterhin hat es sich im Hinblick auf Reduktion der Hautirritation als vorteilhaft herausgestellt, wenn das erfindungsgemäße Mittel frei von Konservierungsmitteln auf Paraben-Basis ist. Parabene sind dem Fachmann als Konservierungsmittel für Lebensmittel wie auch für kosmetische Produkte bekannt. Unter Konservierungsmitteln auf Paraben-Basis sind im Sinne der vorliegenden Anmeldung die verzweigten und unverzweigten C₁-C₁₀-Alkylester der 4-Hydroxybenzoesäure sowie deren physiologisch verträglichen Salze, die sogenannten 4-Hydroxybenzoate, zu verstehen. Insbesondere bevorzugt sind die erfindungsgemäßen Mittel frei von Methyl-4-hydroxybenzoat (Methyl Paraben), Ethyl-4-hydroxybenzoat (Ethyl Paraben), Propyl-4-hydroxybenzoat (Propyl Paraben), 2-Propyl-4-hydroxybenzoat (Isopropyl Paraben), Butyl-4-hydroxybenzoat (Butyl Paraben), 2-Butyl-4-hydroxybenzoat (Isobutyl Paraben) und n-Heptyl-4-hydroxybenzoat (Heptyl Paraben) sowie den Natrium- und/oder Kaliumsalzen von Methyl-4-hydroxybenzoat. In einer bevorzugten Ausführungsform sind die erfindungsgemäßen Mittel daher frei von Konservierungsmitteln auf Paraben-Basis. Frei von Konservierungsmitteln auf Paraben-Basis im Sinne der vorliegenden Erfindung bedeutet, dass die anwendungsbereiten Mittel weniger als 0,05 Gew.-%, bevorzugt weniger als 0,005 Gew.-% und ganz besonders bevorzugt weniger als 0,0001 Gew.-% der Parabene, ausgewählt aus der Gruppe, die gebildet wird aus den verzweigten und unverzweigten C₁-C₁₀-Alkylestern der 4-Hydroxybenzoesäure sowie deren physiologisch verträglichen Salzen, enthält.

Schließlich hat es sich als vorteilhaft herausgestellt, wenn das erfindungsgemäße Mittel frei von Silikonen ist. Unter Silikonen sind im Sinne der vorliegenden Anmeldung flüchtige und nichtflüchtige Silikone zu verstehen. Insbesondere sind die erfindungsgemäßen Mittel frei von Silikonen, die ausgewählt sind aus
(i) Polyalkylsiloxanen (wie Dimethicone), Polyarylsiloxanen, Polyalkylarylsiloxanen, die jeweils flüchtig oder nicht flüchtig, geradkettig, verzweigt oder cyclisch (INCI-Bezeichnung Cyclomethicone), vernetzt oder nicht vernetzt sind;
(ii) Polysiloxanen, die in ihrer allgemeinen Struktur eine oder mehrere organofunktionelle Gruppen enthalten, die ausgewählt sind unter: substituierten oder unsubstituierten aminierten Gruppen (INCI-Bezeichnung: Amodimethicone u/o Trimethylsilylamodimethicone); (per)fluorierten Gruppen; Thiolgruppen; Carboxylatgruppen; hydroxylierten Gruppen (insb. Dimethiconcopolyole); alkoxylierten Gruppen; Acyloxyalkylgruppen; amphoteren Gruppen; Bisulfitgruppen; Hydroxyacylaminogruppen; Carboxygruppen; Sulfonsäuregruppen; und Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikonpolymeren;
(v) oder deren Gemischen.

Solche Silikone werden beispielsweise unter den Handelsnamen: Dow Corning 929 Emulsion (hydroxylamino-modifiziertes Silikon, Amodimethicone), DC 2-2078 (INCI-Bezeichung: Aminopropyl Phenyl Trimethicone), DC 5-7113 (INCI-Bezeichnung: Silicone Quaternium 16), SM-2059 (Hersteller: General Electric), SLM-55067 (Hersteller: Wacker), Abil-Quat 3270 und 3272 (Hersteller: Th. Goldschmidt; diquaternäre Polydimethylsiloxane, Quaternium-80), Q2-7224 (Hersteller: Dow Corning; stabilisiertes Trimethylsilylamodimethicon), DC 8500 (Bis(C₁₃-C₁₅-Alkoxy) PG Amodimethicone), Silcare Silicone SEA (Trideceth-9 PG-Amodimethicone, Fa. Clariant), SILWET (Union Carbide Corporation), Dow Corning 190 (Dimethiconpolyol) und Dow Corning 193 vertrieben. Frei von Silikonen im Sinne der vorliegenden Erfindung bedeutet dabei, dass die anwendungsbereiten Mittel weniger als 0,01 Gew.-%, bevorzugt weniger als 0,005 Gew.-% und besonders bevorzugt weniger als 0,0001 Gew.-% an Silikonen, jeweils bezogen auf das Gesamtgewicht des anwendungsbereiten Mittels, enthalten.

Erfindungsgemäß bevorzugte Mittel sind frei von den UV-Filtersubstanzen, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenzimidazol-5-sulfonsäuren, Benzophenonsulfonsäuren und Benzoylmethanderivaten, frei von Konservierungsmitteln auf Paraben-Basis und/oder frei von Silikonen.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und - distearat sowie PEG-3-distearat, Pigmente, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur Farb- und Formveränderung der Haare sind **dadurch gekennzeichnet, dass** sie einen möglichst neutralen pH-Wert besitzen. Eine weitere bevorzugte Ausführungsform der vorliegenden Erfindung besteht darin, dass das anwendungsbereite Mittel einen pH-Wert zwischen 4,0 und 12,0, bevorzugt zwischen 5,0 und 11,5, insbesondere bevorzugt zwischen 6,0 und 11,0 besitzt. Bei den pH-Werten im Sinne der vorliegenden Erfindung handelt es sich um pH-Werte, die bei einer Temperatur von 22°C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol, Triethanolamin, Ammoniak, 1-Aminopropan-2-ol und 2-Amino-2-methylpropan-1-ol.

Die Konfektionierung der erfindungsgemäßen Farb- und Formveränderungsmittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird; es kann aber bevorzugt sein, die Zubereitung, die zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol enthält, bis zur Anwendung separat zu verpacken.

Erfindungsgemäß bevorzugte Mittel sind **dadurch gekennzeichnet, dass** das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel enthält, welches in einem kosmetischen Träger mindestens ein Oxidationsfarbstoffvorprodukt und zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol enthält, und ein weiterer Container eine Oxidationsmittelzubereitung, enthaltend mindestens ein Oxidationsmittel, enthält. Das Färbemittel besitzt dabei bevorzugt einen pH-Wert zwischen 5,0 und 12,0, bevorzugt zwischen 6,0 und 11,0.

Ein weiterer Gegenstand der vorliegenden Erfindung ist ein Verfahren zur Farb- und/oder Formveränderung keratinischer Fasern, bei dem ein erfindungsgemäßes Mittel gemäß obiger Vorgaben auf das Haar aufgetragen wird, für eine Einwirkzeit von 2 bis 45 min, bevorzugt von 15 bis 30 min auf dem Haar belassen wird, und anschließend das Haar ausgespült wird.

Eine bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt und zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 45 min, bevorzugt von 5 bis 30 min auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45°C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Eine weitere bevorzugte Ausführungsform des erfindungsgemäßen Verfahrens zur dauerhaften Formveränderung keratinischer Fasern liegt vor, wenn eine Zusammensetzung in einem kosmetischen Träger, enthaltend mindestens eine keratinreduzierende Substanz sowie zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol auf das Haar aufgebracht wird, für eine Einwirkzeit von 2 bis 45 min, bevorzugt von 15 bis 35 min auf dem Haar belassen wird, und anschließend gegebenenfalls das Haar ausgespült wird. Anschließend wird ein Fixiermittel, enthaltend mindestens ein Oxidationsmittel, vorzugsweise Wasserstoffperoxid, sowie vorzugsweise zusätzlich mindestens einen Stabilisator oder Komplexbildner, auf das Haar aufgebracht, für eine Einwirkzeit von 2 bis 45 min, bevorzugt von 15 bis 35 min auf dem Haar belassen, und anschließend das Haar ausgespült wird. Die Anwendungstemperaturen bei der erfindungsgemäßen dauerhaften Formveränderung keratinischer Fasern können in einem Bereich zwischen 15 und 45°C liegen. Nach einer Einwirkungszeit wird das Fixiermittel durch Ausspülen von dem Haar entfernt. Das Nachwaschen mit einem Shampoo kann entfallen, wenn ein stark tensidhaltiger Träger verwendet wurde.

Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Das anwendungsbereite Mittel wird bei solchen Systemen vom Anwender oder Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt voneinander konfektionierte Container, wobei ein Container in einem kosmetischen Träger mindestens eine farb- und/oder formverändernde Verbindung und zusätzlich mindestens einen Ester (a) einer C₁₀-C₂₀-Fettsäure mit einem C₆-C₂₂-Fettalkohol sowie mindestens einen Ester (b) einer C₁₀-C₂₀-Fettsäure mit einem C₆-C₁₂-Alkylpolyol enthält, und ein Container eine Oxidationsmittelzusammensetzung, enthaltend mindestens ein chemisches Oxidationsmittel, insbesondere Wasserstoffperoxid, enthält.

Eine bevorzugte Ausführungsform dieses Gegenstand der vorliegenden Erfindung ist eine Mehrkomponentenverpackungseinheit (Kit-of-Parts), enthaltend mindestens zwei getrennt voneinander konfektionierte Container, wobei ein Container eine Färbemischung in einem kosmetischen Träger, enthaltend mindestens eine farbgebende Komponente, insbesondere mindestens ein Oxidationsfarbstoffvorprodukt, sowie zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol, enthält und ein zweiter Container eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Eine weitere bevorzugte Ausführungsform des Erfindungsgegenstands ist eine Mehrkomponentenverpackungseinheit (Kit-of-Parts), enthaltend mindestens zwei getrennt voneinander konfektionierte Container, wobei ein Container eine Formveränderungsmischung in einem kosmetischen Träger, enthaltend mindestens eine formverändernde Komponente und zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol, enthält und ein zweiter Container eine Oxidationsmittelzusammensetzung, enthaltend mindestens ein chemisches Oxidationsmittel, insbesondere Wasserstoffperoxid, enthält.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zur Verringerung der Schädigung der Haarstruktur bei der Farb- und/oder dauerhaften Formveränderung menschlicher Haare.

Ein weiterer Gegenstand der Erfindung ist schließlich die Verwendung der Mittel des ersten Erfindungsgegenstandes zur Verbesserung der Hautverträglichkeit bei der Farb- und/oder dauerhaften Formveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele erläutern die vorliegende Erfindung, ohne sie auf diesen Umfang einzuschränken.

### Beispiele

### 1) Färbecremes

| Rohstoff | E1 | E2 | E3 | E4 | E5 | E6 | E7 | E8 |
|---|---|---|---|---|---|---|---|---|
| Lanette D [1] | 6,60 | 6,60 | 6,60 | 6,60 | 6,60 | 5,50 | -- | -- |
| Lorol techn. [2] | 2,40 | 2,40 | 2,40 | 2,40 | 2,43 | 2,00 | -- | -- |
| Ammonium Carbomer, 1% | -- | -- | -- | -- | -- | -- | 12,00 | 12,00 |
| Lanette E, Pulver [3] | -- | -- | -- | -- | -- | -- | 0,56 | 0,56 |
| Texapon NSF, 27% [4] | -- | -- | -- | -- | -- | -- | 3,52 | 3,52 |
| Kalium Oleat, 12,5% | -- | -- | -- | -- | -- | -- | 2,40 | 2,40 |
| Cutina GMS SE [5] | -- | -- | -- | -- | -- | -- | 1,60 | 1,60 |
| Cutina AGS [6] | -- | -- | -- | -- | -- | -- | 1,60 | 1,60 |
| Eutanol G [7] | -- | -- | -- | -- | -- | -- | 1,60 | 1,60 |
| Hydrenol D [8] | -- | -- | -- | -- | -- | -- | 9,60 | 9,60 |
| Phospholipid EFA [9] | -- | -- | -- | -- | -- | -- | 0,10 | 0,10 |
| Eumulgin B 2 [10] | 0,60 | 0,60 | 0,60 | 0,60 | 0,60 | 0,50 | 2,40 | 2,40 |
| Eumulgin B 1 [11] | 0,60 | 0,60 | 0,60 | 0,60 | 0,60 | 0,50 | -- | -- |
| Lamesoft PO 65 [12] | 2,00 | 2,00 | 2,00 | 2,00 | 2,00 | 2,00 | -- | -- |
| Akypo Soft 45HP [13] | 10,00 | 10,00 | 10,00 | 10,00 | 10,00 | 10,00 | -- | -- |
| Texapon K 14 S Special, 70 % [14] | 2,80 | 2,80 | 2,80 | 2,80 | 2,80 | 2,80 | 1,38 | 1,38 |
| Produkt W 37194 [15] | 3,75 | 3,75 | 3,75 | 3,75 | 3,75 | 3,75 | -- | -- |
| p-Toluylendiaminsulfat | 0,300 | 0,822 | 0,029 | -- | 1,533 | 1,161 | -- | -- |
| Resorcin | 0,350 | 0,416 | 0,011 | -- | 0,410 | 0,298 | 0,53 | 0,53 |
| 2-Methylresorcin | 0,246 | -- | -- | 0,004 | 0,343 | 0,128 | -- | -- |
| 5-Amino-2-methylphenol | -- | 0,023 | 0,001 | -- | -- | -- | -- | -- |
| 2-Amino-4-hydroxyethylamino-anisolsulfat | -- | -- | -- | -- | -- | -- | 0,02 | 0,02 |
| 4-Amino-3-methylphenol | 0,460 | 0,019 | -- | -- | -- | -- | -- | -- |
| 2-Chlor-6-methyl-3-aminophenol | -- | 0,047 | -- | -- | -- | -- | -- | -- |
| Bis-(5-Amino-2-hydroxyphenyl)methan 2HCl | -- | 0,539 | -- | 0,010 | -- | -- | -- | -- |
| 2,4-Diaminophenoxyethanol 2HCl | -- | -- | 0,002 | -- | -- | -- | -- | -- |
| 3-Aminophenol | -- | -- | 0,002 | -- | -- | 0,058 | 0,16 | 0,16 |
| 2,7-Dihydroxynaphthalin | -- | -- | -- | 0,104 | -- | -- | -- | -- |
| 2,4,5,6-Tetraaminopyrimidinsulfat | -- | -- | -- | 0,146 | -- | -- | -- | -- |
| 2-Amino-3-hydroxypyridin | -- | -- | -- | -- | 0,046 | 0,078 | -- | -- |
| Aerosil 200 [16] | -- | -- | -- | -- | -- | -- | 0,25 | 0,25 |
| Ammoniumsulfat, techn. rein | 0,82 | 0,51 | 1,00 | 0,92 | -- | 0,50 | -- | -- |
| Natriumsulfit, wasserfrei, 96% | 0,40 | 0,40 | 0,40 | 0,40 | 0,40 | 0,40 | 0,20 | 0,20 |
| HEDP, 60% | 0,20 | 0,20 | 0,20 | 0,20 | -- | 0,20 | -- | -- |
| Na₄-EDTA, Pulver, 87% | -- | -- | -- | -- | -- | -- | 0,20 | 0,20 |
| Natriumhydroxid, 45% | 1,00 | 1,12 | 0,10 | 0,30 | 1,70 | -- | -- | -- |
| Kaliumhydroxid, 50% | -- | -- | -- | -- | -- | 0,90 | 0,85 | 0,85 |
| Ascorbinsäure | -- | -- | -- | -- | -- | 0,05 | 0,05 | 0,05 |
| Natriumsilikat 40/42 [17] | 0,50 | 0,50 | 0,50 | 0,50 | 0,50 | 0,50 | -- | -- |
| Hydrovance [18] | -- | -- | -- | -- | -- | -- | 2,00 | 2,00 |
| L-Serin | 1,00 | 1,00 | 1,00 | 1,00 | 1,00 | -- | 0,50 | 0,50 |
| Olivem 1000 Crystal Skin [19] | 2,00 | 1,00 | 1,00 | 2,00 | 1,00 | 1,00 | 4,00 | 2,00 |
| Ronacare Ectoin [20] | -- | 0,50 | 1,00 | -- | 0,50 | 1,00 | -- | 0,50 |
| Ammoniak, 25 % | 7,00 | 6,50 | 7,00 | 7,00 | 6,50 | 7,50 | 6,00 | 6,00 |
| Parfum | qs | qs | qs | qs | qs | qs | qs | qs |
| Wasser, entsalzt | add 100 | | | | | | | |

| | | | | | | | | |
|---|---|---|---|---|---|---|---|---|
| [1] C₁₆-C₁₈-Fettalkohol (INCI-Bezeichnung: Cetearyl alcohol) (Cognis) [2] C₁₂-C₁₈-Fettalkohol (INCI-Bezeichnung: Coconut alcohol) (Cognis) [3] C₁₆-C₁₈-Fettalkoholsulfat, Natrium-Salz (INCI-Bezeichnung: Sodium Cetearyl Sulfate) (Cognis) [4] C₁₂-Fettalkoholsulfat, ethoxyliert (2 EO) Natrium-Salz (INCI-Bezeichnung: Sodium Laureth Sulfate) (Cognis) [5] Stearinsäureglycerylester (INCI-Bezeichnung: Glyceryl Stearate) (Cognis) [6] Ethylenglycoldistearylester (INCI-Bezeichnung: Glycol Distearate) (Cognis) [7] 2-Octyldodecanol (INCI-Bezeichnung: Octyldodecanol) (Cognis) [8] C₁₆-C₁₈-Fettalkohol (INCI-Bezeichnung: Cetearyl alcohol) (Cognis) [9] zwitterionisches Phospholid (INCI-Bezeichnung: Linoleamidopropyl PG-dimonium chloride phosphate) (Uniqema) [10] C₁₆-C₁₈-Fettalkohol, ethoxyliert (20 EO) (INCI-Bezeichnung: Ceteareth-20) (Cognis) [11] C₁₆-C₁₈-Fettalkohol, ethoxyliert (12 EO) (INCI-Bezeichnung: Ceteareth-12) (Cognis) [12] C₁₂-C₁₈-Alkylpolyglucosid, Glycerylmonooleat (ca. 66 %, INCI-Bezeichnung: Coco-Glucoside, Glyceryl Oleate, Aqua) (Cognis) [13] C₁₂-C₁₄-Alkylether, ethoxyliert (6 EO) Carbonsäure, Natriumsalz (ca. 21 %, INCI-Bezeichnung: Sodium Laureth-6 Carboxylate, Aqua) (KAO) [14] C₁₂-C₁₄-Alkylethersulfat, ethoxyliert (3 EO), Natriumsalz (ca. 70 %, INCI-Bezeichnung: Sodium Myreth Sulfate, Aqua) (Cognis) [15] Copolymer aus Acrylsäure, Natriumsalz und Trimethylammoniopropylacrylamidchlorid (ca. 20 %, INCI-Bezeichnung: Acrylamidopropyltrimonium Chloride/Acrylates Copolymer, Aqua) (Stockhausen) [16] Siliciumdioxid, pyrogen (INCI-Bezeichnung: Silica) (Evonik) [17] Natronwasserglas [18] Hydroxyethylharnstoff (ca. 50 %; INCI-Bezeichnung: Hydroxyethylura, Aqua) (National Starch) [19] Fettsäureestergemisch (INCI-Bezeichnung: Cetearyl Olivate, Sorbitan Olivate) (B&TCompany) [20] (*4S*)-1,4,5,6-Tetrahydro-2-methyl-pyrimidin-4-carbonsäure (INCI-Bezeichnung: Ectoin) (Rona) | | | | | | | | |

| Rohstoff | E9 | E10 | E11 | E12 |
|---|---|---|---|---|
| Cetiol V [21] | 2,30 | 2,30 | 2,30 | 2,30 |
| Xanthan FN [22] | 0,05 | 0,05 | 0,05 | 0,05 |
| Lanette N [23] | 14,00 | 14,00 | 14,00 | 14,00 |
| Lanette D | 3,90 | 3,90 | 3,90 | 3,90 |
| Cutina GMS SE | 6,00 | 6,00 | 6,00 | 6,00 |
| Kokosamidopropylbetain, 40 % | 2,00 | 2,00 | 2,00 | 2,00 |
| Monoethanolamin | 0,30 | 0,30 | 0,30 | 0,30 |
| Natriumsulfit wasserfrei, 96% | 0,20 | 0,20 | 0,20 | 0,20 |
| RonaCare Ectoin | 1,00 | 1,00 | -- | -- |
| Olivem 1000 Crystal Skin | 1,00 | 1,00 | 1,00 | 2,00 |
| Ascorbinsäure | 0,05 | 0,05 | 0,05 | 0,05 |
| Ammoniak, 25 % | 6,00 | 6,00 | 6,00 | 6,00 |
| 4,5-Diamino-1-(2-hydroxyethyl)pyrazolsulfat | 1,00 | 1,00 | 1,00 | 1,00 |
| 5-Amino-2-methylphenol | 0,20 | 0,20 | 0,20 | 0,20 |
| 2-Amino-3-hydroxypyridin | 0,20 | 0,20 | 0,20 | 0,20 |
| 2-Amino-6-chloro-4-nitrophenol | 0,30 | -- | 0,30 | -- |
| Wasser, vollentsalzt | add 100 | | | |

| | | | | |
|---|---|---|---|---|
| [21] Ölsäuredecylester (INCI-Bezeichnung: Decyl Oleate) (Cognis) [22] Xanthan Gum (INCI-Bezeichnung: Xanthan Gum) (Jungbunzlauer) [23] C₁₆-C₁₈-Fettalkohol, Natrium C₁₆-C₁₈-Fettalkoholsulfat (INCI-Bezeichnung: Cetearyl alcohol, Sodium Cetearyl Sulfate) (Cognis) | | | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

### 2) Entwicklerzubereitungen

| EW1: | | EW2: | |
|---|---|---|---|
| Rohstoff | Gew.-% | Rohstoff | Gew.-% |
| Dipicolinsäure | 0,10 | Na-benzoat | 0,04 |
| Dinatriumpyrophosphat | 0,03 | Dipicolinsäure | 0,10 |
| HEDP, 60% | 1,50 | Dinatriumpyrophosphat | 0,19 |
| Texapon NSO [24] | 2,00 | 1,2-Propandiol | 0,50 |
| Dow Corning DB 110 A [25] | 0,07 | HEDP, 60% | 0,25 |
| Aculyn 33A [26] | 15,00 | Paraffinum Liquidum | 0,30 |
| Ammoniak, 25 % | 0,65 | Genamin STAC [27] | 0,20 |
| Wasserstoffperoxid, 50 % | 12,00 | Cetearyl Alcohol | 3,00 |
| Wasser, vollentsalzt | ad 100 | Eumulgin B 2 | 0,70 |
| | | Wasserstoffperoxid, 50 % | 12,2 |
| | | Kaliumhydroxid, 50 % | 0,19 |
| | | Wasser | ad 100 |

| | | | |
|---|---|---|---|
| [24] Laurylalkohol-diglykolethersulfat, Na-Salz (ca. 28%, INCI-Bezeichnung: Sodium Laureth Sulfate) (Cognis) [25] Acrylpolymer (ca. 28% in Wasser; INCI-Bezeichnung: Acrylates Copolymer) (Rohm & Haas) [26] nicht-ionische Silikonemulsion (INCI-Bezeichnung: Dimethicon) (Dow Corning) [27] Trimethylstearylammonium chlorid (ca. 80% Aktivsubstanz; INCI-Bezeichnung: Steartrimonium Chloride) (Clariant) | | | |

### 3) Ausfärbungen:

Die Färbecremes E1 bis E6 wurden vor der Anwendung mit einer Entwicklerlösung EW1 im Gewichtsverhältnis von 1:1 versetzt und innig vermischt. Die Färbecremes E7 und E8 wurden vor der Anwendung mit einer Entwicklerlösung EW2 im Gewichtsverhältnis von 1:1 versetzt und innig vermischt. Die Färbecremes E9 bis E12 wurden vor der Anwendung mit einer 6 Gew.-%igen Wasserstoffperoxidzubereitung im Gewichtsverhältnis von 1:2 versetzt und innig vermischt. Pro Gramm Haar (europäisches Humanhaar, Alkinco 6634, #10/2003, A9) wurden 4 g des frisch hergestellten, anwendungsbereiten Färbemittels aufgetragen. Die Einwirkzeit betrug 30 min bei 35°C für die Färbemittel. Danach wurden die Strähnen 30 s lang mit warmem Wasser ausgespült und luftgetrocknet. Die gefärbten Strähnen zeichneten sich durch glänzende Farben und einen angenehmen Griff aus.

### 4) Aufhellendes Haarpfleqeshampoo:

| | | | | |
|---|---|---|---|---|
| Ethoxyliertes Fettalkoholsulfat (C₁₂-C₁₄, 2 EO; 70% AS) | | | 15,0 | |
| Disodium Cocoamphodiacetate | | | 7,0 | |
| Euperlan^{®}PK 3000 AM | [28] | | 2,5 | |
| Coconut Glycerides + 7,3 EO | | | 0,5 | |
| Cutina HR^{®} | | [29] | | 0,1 |
| D-Panthenol | | | | 0,5 |
| Olivem 1000 Crystal Skin | | | | 0,5 |
| Zitronensäure | | | 0,5 | |
| Wasserstoffperoxid | | | 0,2 | |
| Salicylsäure | | | | 0,2 |
| Trinatriumcitrat Dihydrat | | | | 0,2 |
| Sodium Benzoate | | | | 0,4 |
| Glycerin | | | | 0,2 |
| NaCl | | | | 1,0 |
| Parfum | | | qs | |
| Wasser | | | ad 100 | |

### 5) Aufhellende Rinse-off Conditioner:

| | | | | |
|---|---|---|---|---|
| Cetearyl Alcohol | | | | 2,5 |
| Eumulgin^{®} B2 | | | 0,5 | |
| Genamin KDMP^{®} | | [30] | | 3,0 |
| Cutina CP^{®} | | [31] | | 0,5 |
| Natrosol 250 HR^{®} | | [32] | | 0,5 |
| Timiron Super Silver^{®} | [33] | | 0,1 | |
| Mannitol | | | | 0,1 |
| Glycin | | | | 0,2 |
| Methylparaben | | | 0,3 | |
| Phenoxyethanol | | | | 0,4 |
| D-Panthenol | | | | 0,5 |
| Olivem 1000 Crystal Skin | | | 1,0 | |
| Wasserstoffperoxid | | | 0,4 | |
| Citronensäure | | | 0,2 | |
| Parfum | | | qs | |
| Wasser | | | ad 100 | |

Es wurden die folgenden Handelsprodukte eingesetzt:
[28] INCI-Bezeichnung: Aqua, Glycol Distearate, Glycerin, Laureth-4, Cocamidopropyl Betaine, Formic Acid (Solids: 41-45%); Cognis
[29] INCI-Bezeichnung: Hydrogenated Castor Oil; Cognis
[30] INCI-Bezeichnung: Behentrimonium Chloride; Cognis
[31] INCI-Bezeichnung: Cetyl Palmitate; Cognis
[32] INCI-Bezeichnung: Hydroxyethylcellulose; Hercules
[33] INCI-Bezeichnung: Mica; CI 77891; Merck

## Patentansprüche

1. Mittel zur Farb- und/oder Formveränderung von keratinischen Fasern, insbesondere menschlichen Haaren, enthaltend in einem kosmetischen Träger mindestens eine farb- und/oder formverändernde Komponente, **dadurch gekennzeichnet, dass** das Mittel zusätzlich mindestens einen Ester (a) einer C₁₀-C₂₀-Fettsäure mit einem C₆-C₂₂-Fettalkohol und mindestens einen Ester (b) einer C₁₀-C₂₀-Fettsäure mit einem C₆-C₁₂-Alkylpolyol enthält.

2. Mittel nach Anspruch 1, **dadurch gekennzeichnet, dass** die Ester (a) und (b) jeweils ein Gemisch aus Estern von mindestens einer ungesättigten C₁₀-C₂₀-Fettsäure sowie mindestens einer gesättigten C₁₀-C₂₀-Fettsäure ist.

3. Mittel nach einem der Ansprüche 1 bis 2, **dadurch gekennzeichnet, dass** das Mittel als Ester (a) und (b) jeweils ein Gemisch aus Estern von C₁₀-C₂₀-Fettsäuren, welches aus Olivenöl gewonnen wird, enthält.

4. Mittel nach einem der Ansprüche 1 bis 3, **dadurch gekennzeichnet, dass** das Mittel als Ester (a) mindestens einen Ester einer C₁₀-C₂₀-Fettsäure mit einem C₆-C₂₂-Fettalkohol enthält, worin der besagte Fettalkohol ausgewählt ist aus der Gruppe, die gebildet wird aus Capronalkohol, Caprylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoleylalkohol, Linolenylalkohol, Elaeostearylalkohol, Arachylalkohol, Gadoleylalkohol, Behenylalkohol, Erucylalkohol und Brassidylalkohol sowie den Fettalkoholgemischen Talgfettalkohol, Cocosfettalkohol, Palmfettalkohol und Cetearylalkohol.

5. Mittel nach einem der Ansprüche 1 bis 4, **dadurch gekennzeichnet, dass** das Mittel als Ester (b) mindestens ein Ester einer C₁₀-C₂₀-Fettsäure mit einem C₆-C₁₂-Alkylpolyol enthält, worin der besagte Polyol ausgewählt ist aus der Gruppe, die gebildet wird aus den Hexitolen Galactitol, Mannitol und Sorbitol sowie aus Sorbitan.

6. Mittel nach Anspruch 5, **dadurch gekennzeichnet, dass** das Mittel als Ester ein Gemisch aus mindestens Cetearylolivat und mindestens Sorbitanolivat enthält.

7. Mittel einem der Ansprüche 1 bis 6, **dadurch gekennzeichnet, dass** das Mittel das Gemisch der Ester aus C₁₀-C₂₀-Fettsäure(n) mit C₆-C₂₂-Fettalkohol(en) sowie mit C₆-C₁₂-Alkylpolyol(en) in einer Gesamtmenge von 0,0001 bis 5 Gew.-%, insbesondere von 0,001 bis 3,0 Gew.-%, jeweils bezogen auf das Gesamtgewicht des anwendungsbereiten Mittels, enthält.

8. Mittel nach einem der Ansprüche 1 bis 7, **dadurch gekennzeichnet, dass** das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens Oxofarbstoffvorprodukte und/oder mindestens ein Aufhellmittel enthält.

9. Mittel nach Anspruch 8, **dadurch gekennzeichnet, dass** es als farbverändernde Komponente mindestens ein Oxidationsfarbstoffvorprodukt enthält, wobei der Gewichtsanteil aller Oxidationsfarbstoffvorprodukte zwischen 2,0 und 25,0 Gew.-%, bevorzugt zwischen 3,0 und 20,0 Gew.-% und insbesondere zwischen 4,0 und 15,0 Gew.-%, jeweils bezogen auf das Gesamtgewicht des anwendungsbereiten Mittels, liegt.

10. Mittel nach einem der Ansprüche 1 bis 9, **dadurch gekennzeichnet, dass** das Mittel zusätzlich ein Oxidationsmittel, ausgewählt aus Wasserstoffperoxid sowie seinen festen Anlagerungsprodukten an organische und anorganische Verbindungen, enthält.

11. Mittel nach einem der Ansprüche 1 bis 10, **dadurch gekennzeichnet, dass** das das Mittel als zusätzlichen Haarpflegestoff mindestens einen Pflegestoff, ausgewählt aus der Gruppe, gebildet aus D-Panthenol, Glycin, Serin, Arginin, Moringa Oleifera und/oder Ectoin, enthält.

12. Mittel nach einem der Ansprüche 1 bis 11, **dadurch gekennzeichnet, dass** es frei ist von UV-Filtersubstanzen, und/oder frei von Konservierungsmitteln auf Paraben-Basis ist, und/oder frei von Silikonen ist.

13. Verwendung eines Mittels gemäß einem der Ansprüche 1 bis 12 zur Verbesserung der Hautverträglichkeit bei der Farb- und/oder dauerhaften Formveränderung menschlicher Haare.

14. Verwendung eines Mittels gemäß einem der Ansprüche 1 bis 12 zur Verringerung der Schädigung der Haarstruktur bei der Farb- und/oder dauerhaften Formveränderung menschlicher Haare.

15. Kit-of-Parts, enthaltend mindestens zwei getrennt konfektionierte Container, wobei ein Container eine farb- und/oder formverändernde Verbindung gemäß einem der Ansprüche 1 bis 12 enthält, und ein Container eine Oxidationsmittelzusammensetzung, enthaltend mindestens ein chemisches Oxidationsmittel, insbesondere Wasserstoffperoxid, enthält.
